# EUROPEAN PATENT APPLICATION

(11) **EP 4 501 909 A1**
(43) Date of publication of application: **05.02.2025**
(21) Application number: 23780166.7
(22) Date of filing: 24.03.2023
(51) Int. Cl.: C07D 207/16, C07F 15/02

(54) **HETEROCYCLE-CONTAINING AMINO ACID COMPOUND AND COMPLEX**

(30) Priority: 30.03.2022 JP 2022054884
(71) Applicant: Aichi Steel Corporation, Tokai-shi, Aichi 476-8666 (JP); Tokushima University, Tokushima-shi, Tokushima 770-8501 (JP)
(72) Inventor: MERA Akane, Tokai-shi, Aichi 476-8666 (JP); SUZUKI Motofumi, Tokai-shi, Aichi 476-8666 (JP); NAMBA Kosuke, Tokushima-shi, Tokushima 770-8501 (JP)
(74) Representative: TBK
(86) International application number: PCT/JP2023/011815
(87) International publication number: WO 2023/190156

(57) **Abstract**

The present compound is a heterocycle-containing amino acid compound represented by a general formula (1) or a salt thereof. In the formula (1), n is 1, 2, or 3, and R¹ represents a hydrogen atom, -OH, -NH₂, -NH-CO-CH₃, -NH-CO-CH₂OH,or -NR²-(CH₂)ₘ-COOH, where m is 1 or 2, and R² represents a hydrogen atom or a methyl group. The present complex includes the heterocycle-containing amino acid compound or a salt thereof and a metal element.

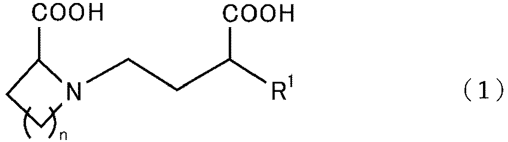

## Description

### [Field of the Invention]

The present invention relates to a novel heterocycle-containing amino acid compound and a complex.

### [Background Art]

Various trace metal elements are involved in the growth of plants and the maintenance of their functions. When the trace metal elements are deficient, the plants cannot glow normally. Iron is an element necessary for respiration, photosynthesis, DNA synthesis, and the like, and, particularly, is an active central metal of an enzyme essential for chlorophyll biosynthesis. Accordingly, iron deficiency causes chlorosis (iron-deficiency chlorosis) which involves yellowing of leaves.

Meanwhile, a poor soil regarded as an unsuitable soil for agriculture accounts for about 67% of the total land area in the world, and a half thereof is an alkaline soil. In such alkaline soil, iron exists in the form of trivalent ferric hydroxide (Fe(OH)₃), which is insoluble in water. Plants cannot sufficiently absorb iron from the roots, leading to iron deficiency.

As a method of growing plants, hydroponic cultivation is known in which plants are grown using a culture solution prepared by dissolving nutrients in water (hereinafter, referred to as "hydroponic nutrient solution") without using soil. The nutrients contained in the hydroponic nutrient solution include macronutrients such as nitrogen, phosphorus, and potassium; and micronutrients such as manganese, zinc, iron, and molybdenum. Iron of the nutrients is contained as an iron complex, such as Fe-EDTA and Fe-DTPA, to enhance solubility in water.

In hydroponic cultivation, the hydroponic nutrient solution may be used while being circulated. In this case, the hydroponic nutrient solution may be sterilized by, for example, heating or ultraviolet irradiation, to prevent the growth of pathogenic bacteria. In particular, the ultraviolet irradiation method is widely adopted because a high sterilization effect can be obtained in a short time.

### [Prior Art Literature]

### [Patent Literature]

Patent Literature 1: JP 2016-63758 A

### [Summary of the Invention]

### [Problems that the Invention is to Solve]

An object of the present invention is to provide a heterocycle-containing amino acid compound which has an ability to uptake a metal such as iron and easily forms a complex, and a complex suitable for plant cultivation which is prepared using the heterocycle-containing amino acid compound.

There are some iron complexes which absorb the ultraviolet rays and are decomposed, or then precipitated in the case where the complexes are irradiated with ultraviolet rays. Accordingly, when the liquid fertilizer containing such a complex is circulated while being irradiated with ultraviolet rays, the following problems are caused. The circulation efficiency is decreased due to the precipitation, and grown plants have iron deficiency due to a change in composition of the liquid fertilizer.

Another object of the present invention is to provide a complex which is stable because decomposition is suppressed when a solution thereof is irradiated with ultraviolet rays, and which is suitable for plant cultivation using a liquid fertilizer containing the solution.

### [Means for Solving the Problems]

The present invention is as follows.
[1] A heterocycle-containing amino acid compound represented by a general formula (1) or a salt thereof. 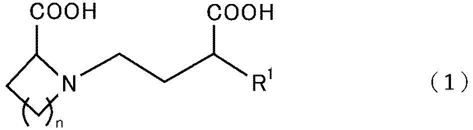 (In the formula, n is 1, 2, or 3, and R¹ represents a hydrogen atom, -OH, -NH₂, -NH-CO-CH₃, -NH-CO-CH₂OH, or -NR²-(CH₂)ₘ-COOH, where m is 1 or 2, and R² represents a hydrogen atom or a methyl group.)
[2] The heterocycle-containing amino acid compound or a salt thereof according to [1] above, wherein the heterocycle-containing amino acid compound is a compound represented by a general formula (2) or (3), 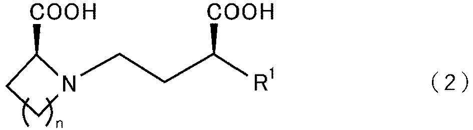 (In the formula, n is 1, 2, or 3, and R¹ represents -OH, -NH₂, -NH-CO-CH₃, -NH-CO-CH₂OH, or -NR²-(CH₂)ₘ-COOH, where m is 1 or 2, and R² represents a hydrogen atom or a methyl group.) 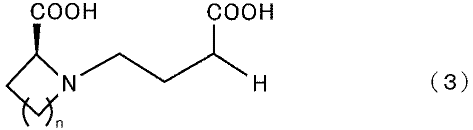 (In the formula, n is 1, 2 or 3.)
[3] A complex comprising: the heterocycle-containing amino acid compound or a salt thereof according to [1] or [2] above; and a metal element.
[4] The complex according to [3] above, wherein the metal element is Fe.

### [Effects of the Invention]

When the heterocycle-containing amino acid compound of the present invention is used, an iron complex, for example, can be easily formed.

The complex of the present invention is suitable for plant cultivation by soil cultivation and hydroponic cultivation. The complex of the present invention is suitable for soil cultivation, particularly plant cultivation using alkaline soil. The complex of the present invention is stable due to suppressed decomposition when a solution of the complex is irradiated with ultraviolet rays. Thus, the complex is suitable as a raw material for a liquid fertilizer, such as a hydroponic nutrient solution to be subjected to the ultraviolet irradiation method for sterilization in hydroponic cultivation.

### [Brief Description of Drawings]

Fig. 1 is a graph showing measurement results of leaf color (SPAD value) of seedlings when corn is hydroponically cultivated using compounds V2 and V7.
Fig. 2 is a graph showing measurement results of leaf color (SPAD value) of seedlings when rice is cultivated in alkaline soil using compounds V1, V3, and V4.
Fig. 3 is a graph showing measurement results of leaf color (SPAD value) of seedlings when rice is cultivated in alkaline soil using compounds V2, V5, V6, and V7.

### [Embodiment for Carrying out the Invention]

The heterocycle-containing amino acid compound of the present invention is a compound represented by the following general formula (1) or a salt thereof. 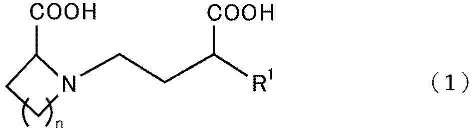 (In the formula, n is 1, 2, or 3, and R¹ represents a hydrogen atom, -OH, -NH₂, -NH-CO-CH₃, -NH-CO-CH₂OH, or -NR²-(CH₂)ₘ-COOH, where m is 1 or 2, and R² represents a hydrogen atom or a methyl group.)

In the present invention, the heterocycle-containing amino acid compound and a salt thereof may have an isomer such as an optical isomer, a stereoisomer, and a positional isomer. For example, when the heterocycle-containing amino acid compound and a salt thereof have an optical isomer, an optical isomer divided from a racemic body is also included in the heterocycle-containing amino acid compound and a salt thereof in the present invention.

Preferable examples of the optical isomer for the heterocycle-containing amino acid compound or a salt thereof in the present invention include compounds represented by the following general formulae (2) and (3) or salts thereof. 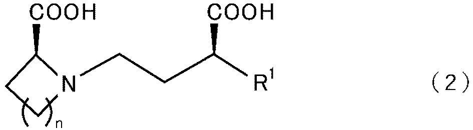 (In the formula, n is 1, 2, or 3, and R¹ represents -OH, -NH₂, -NH-CO-CH₃, -NH-CO-CH₂OH, or -NR²-(CH₂)ₘ-COOH, where m is 1 or 2, and R² represents a hydrogen atom or a methyl group.)

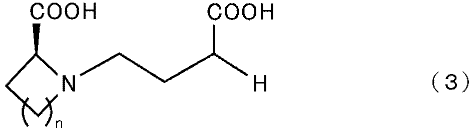

### (In the formula, n is 1, 2 or 3.)

The heterocycle-containing amino acid compound of the present invention is easily soluble in water and suitable for complex formation.

The production method of the heterocycle-containing amino acid compound in the present invention is not particularly limited. Hereinafter, a preferred non-limiting method for producing a compound having a 5-membered ring will be described.

A 5-membered ring compound which is a salt of the heterocycle-containing amino acid compound represented by the above general formula (1) and in which R¹ represents a hydrogen atom (hereinafter, referred to as "heterocycle-containing amino acid compound (H1)") can be produced, for example, by reacting an L-proline alkyl ester (such as L-proline tert-butyl) with an alkyl 4-oxobutanoate (such as tert-butyl 4-oxobutanoate) in a solvent in the presence of a reducing agent, then appropriately subjecting the resultant solution to extraction, purification, and the like to form a precursor having a group (in this case, an alkyl group derived from a tert-butyl group) serving as a protecting group for a carboxy group, and then deprotecting the alkyl group.

Examples of the solvent include an alcohol such as methanol, ethanol, isopropanol, and ethylene glycol; an aprotic polar solvent such as acetonitrile, dimethylformamide (DMF), and dimethylsulfoxide (DMSO); and the like. These solvents may be used singly or in combination of two or more types thereof.

Examples of the reducing agent include a boron compound such as sodium cyanoborohydride and sodium triacetoxyborohydride.

When the deprotection is carried out, inorganic acids such as hydrogen chloride, hydrogen bromide, hydrogen fluoride, hydrogen iodide, aluminum chloride, aluminum bromide, boron trichloride, boron tribromide, sulfuric acid, and phosphoric acid and organic acids such as formic acid, acetic acid, trifluoroacetic acid, propionic acid, methanesulfonic acid, p-toluenesulfonic acid, and trifluoromethanesulfonic acid may be used as a deprotecting agent. The deprotection may be performed in a solvent.

A 5-membered ring compound which is the heterocycle-containing amino acid compound represented by the above general formula (1) and in which R¹ is -OH (hydroxy group) can be produced, for example, by reacting L-proline tert-butyl with a reactive aldehyde having an aldehyde group on one end of the molecule and having an ester bond containing a saturated hydrocarbon group and an alkoxy group on the other end in a solvent in the presence of a reducing agent (reductive amination reaction), and then appropriately subjecting the resultant solution to extraction, purification, and the like to form a precursor having a group (an alkyl group, in this case, a tert-butyl group) serving as a protecting group for a carboxy group and an alkoxy group derived from the reactive aldehyde, and then deprotecting the tert-butyl group from the precursor. The solvent, the reducing agent, and the deprotecting agent similar to those described for the heterocycle-containing amino acid compound (H1) may be used.

A 5-membered ring compound which is a heterocycle-containing amino acid compound represented by the above general formula (1) and in which R¹ is -NH₂ (amino group) can be produced, for example, by oxidatively cleaving a vinyl group of a compound represented by CH₂=CHCH₂CH(COOR²¹)NHR²³ (R²¹ represents a protecting group for a carboxy group, and R²³ represents a protecting group for an amino group) to form an aldehyde, then subjecting the aldehyde and L-proline to an amination reaction in the presence of a reducing agent, subjecting the resultant reaction product to a dehydration condensation reaction with an alcohol to form a first precursor in which a carboxy group derived from L-proline is protected, then deprotecting a protecting group for an amino group in the first precursor by catalytic reduction, and deprotecting a protecting group for a carboxy group in the resultant second precursor (see general formula (11) below): 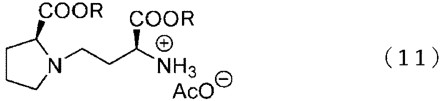 (In the formula, Rs may be identical with or different from each other and each represent a saturated hydrocarbon group.)

Examples of R²¹ in the raw material CH₂=CHCH₂CH(COOR²¹)NHR²³ include a linear, branched or cyclic alkyl group having 1-6 carbon atoms, such as methyl group, ethyl group, n-propyl group, iso-propyl group, n-butyl group, isobutyl group, tert-butyl group, n-hexyl group, and cyclohexyl group; an aralkyl group which may have a substituent, such as benzyl group, p-nitrobenzyl group, o-nitrobenzyl group, m-nitrobenzyl group, 2,4-dinitrobenzyl group, p-chlorobenzyl group, p-bromobenzyl group, and p-methoxybenzyl group; an alkylcarbonyloxy-alkyl group, the alkylcarbonyloxy having 1-6 carbon atoms, such as acetoxymethyl group, acetoxyethyl group, propionyloxymethyl group, n-butyryloxymethyl group, isobutyryloxymethyl group, and pivaloyloxymethyl group; and the like. Examples of R²³ include include an alkoxycarbonyl group which may be substituted with halogen, such as methoxycarbonyl group, ethoxycarbonyl group, 2,2,2-trichloroethoxycarbonyl group, and tert-butoxycarbonyl group (Boc); an alkenyloxycarbonyl group such as vinyloxycarbonyl group; an aralkyloxycarbonyl group such as benzyloxycarbonyl group (Cbz) and 9-fluorenylmethoxycarbonyl group; an aralkyl group which may have a substituent, such as benzyl group, p-nitrobenzyl group, o-nitrobenzyl group, m-nitrobenzyl group, 2,4-dinitrobenzyl group, p-chlorobenzyl group, p-bromobenzyl group, and p-methoxybenzyl group; an acyl group such as formyl group, acetyl group, trifluoroacetyl group, and benzoyl group; an arylsulfonyl group such as p-toluenesulfonyl group and benzenesulfonyl group; an alkylsulfonyl group such as methanesulfonyl group; and the like.

The reducing agent and the deprotecting agent for deprotecting the protecting group of the carboxy group similar to those described for the heterocycle-containing amino acid compound (H1) may be used.

In the catalytic reduction for deprotecting a protecting group of an amino group, a method for performing hydrogenolysis using a transition metal catalyst such as Pd, Pt, Ru, and Rh; a method for performing hydrogenolysis using a catalyst on which a transition metal such as Pd-carbon and palladium hydroxide-carbon (Pearlman's catalyst) is supported; Birch reduction method; and the like may be applied.

A 5-membered ring compound which is a heterocycle-containing amino acid compound represented by the above general formula (1) and in which R¹ is -NHCOCH₃ (acetamide group) can be produced, for example, by adding acetic anhydride to a solution prepared by dissolving a compound represented by the above general formula (11) in a solvent in the presence of an organic base such as triethylamine, reacting the resultant mixture to convert -NH₃⁺·AcO⁻ to -NHCOCH₃, and then deprotecting the resultant reaction solution to convert -COOR derived from the above general formula (11) to -COOH.

The deprotecting agent similar to that described for the heterocycle-containing amino acid compound (H1) may be used.

A 5-membered ring compound which is a heterocycle-containing amino acid compound represented by the above general formula (1) and in which R¹ is -NHCOCH₂OH can be produced, for example, by adding a compound having a group serving as a protecting group for a hydroxy group (such as tert-butyldimethylsilyl glycolate (HOCH(O)CH₂-O-TBS)) to a solution prepared by dissolving a compound represented by the above general formula (11) in a solvent in the presence of an organic base (such as N,N-dimethyl-4-aminopyridine) and an activating agent for a carboxy group (such as 1 -ethyl-3-(3-dimethylaminopropyl)carbodiimide hydrochloride (EDCI)), and reacting the resultant mixture to convert -NH₃⁺·AcO⁻ to -NHCOCH₂-O-TBS, then deprotecting the resultant reaction solution to convert -O-TBS to -OH, and further converting -COOR derived from the above general formula (11) to -COOH.

The deprotecting agent similar to that described for the heterocycle-containing amino acid compound (H1) may be used.

A 5-membered ring compound which is a heterocycle-containing amino acid compound represented by the above general formula (1) and in which R¹ is -NHCH₂COOH can be produced, for example, by adding an alkyl glyoxylate (such as ethyl glyoxylate) as a reactive aldehyde to a solution prepared by dissolving a compound represented by the above general formula (11) in a solvent in the presence of a reducing agent, then reacting the resultant mixture to convert -NH₃⁺·AcO⁻ to -NHCH₂COOEt (in the case of ethyl glyoxylate), then deprotecting the resultant reaction solution to convert -OEt to -OH, and further converting -COOR derived from the above general formula (11) to -COOH.

The reducing agent and the deprotecting agent similar to those described for the heterocycle-containing amino acid compound (H1) may be used.

A 5-membered ring compound which is a heterocycle-containing amino acid compound represented by the above general formula (1) and in which R¹ is -N(CH₃)-(CH₂)₂COOH can be produced, for example, by reacting a compound having a carbon-carbon double bond on one end of the molecule and an ester bond containing a saturated hydrocarbon group on the other end with an oxidizer (such as ozone gas) in a solvent to convert the carbon-carbon double bond moiety to an aldehyde group, reacting the aldehyde compound with a compound represented by the above general formula (11) to convert -NH₃⁺·AcO⁻ to -N(CH₃)-(CH)₂COOR, then deprotecting the resultant reaction solution to convert -(CH)₂COOR to -(CH)₂COOH, and further converting -COOR derived from the above general formula (11) to -COOH.

The complex of the present invention is a metal complex containing the heterocycle-containing amino acid compound and a metal element. Specifically, the complex of the present invention is a metal complex in which a ligand consisting of the heterocycle-containing amino acid compound is coordinate-bonded to a central atom based on the metal element. In the present invention, the number of ligands for one central atom is not particularly limited, and may be one or two or more. A complex having two ligands has a dimer structure.

In the complex of the present invention, the metal element may be, for example, Fe, Zn, Cu, Mn, Ca, Mg, Ni, Cd, Pb, Co, or Al. Among these elements, Fe is preferable because the complex is suitable for plant cultivation.

The complex of the present invention can be produced by mixing an aqueous solution of the heterocycle-containing amino acid compound with a compound having a metal element that forms a central atom or an aqueous solution of the compound, and adjusting the pH to a predetermined value (e.g., pH1 to pH10). Examples of the compound having a metal element include sulfates, sulfites, nitrates, nitrites, phosphates, carbonates, halides, perhalogenates, hypohalites, oxides, and the like.

When the central element of the complex is Fe, iron sulfate, iron nitrate, iron nitrite, iron chloride, iron perchlorate or the like may be used as a raw material for production.

When the complex of the present invention is produced by the above method, an aqueous solution containing the complex is normally obtained. Particularly, an aqueous solution containing an Fe complex or a mixed solution prepared by further adding an additional component (such as a macronutrient) to the aqueous solution is preferably used as a liquid fertilizer for plant cultivation by soil cultivation and hydroponic cultivation. The pH (25°C) of the liquid fertilizer is preferably in a range from 1 to 10.

In hydroponic cultivation, when a hydroponic nutrient solution as a liquid fertilizer is used while being circulated, the hydroponic nutrient solution is sometimes sterilized with ultraviolet rays to prevent the growth of pathogenic bacteria. The ultraviolet rays are light having a wavelength in a range of 10 to 400 nm. When the liquid fertilizer containing the complex of the present invention is used as the hydroponic nutrient solution and the hydroponic nutrient solution is irradiated with such ultraviolet rays, decomposition of the complex is suppressed. Specifically, precipitation formation in the hydroponic nutrient solution after ultraviolet irradiation is suppressed, and a change in composition of the hydroponic nutrient solution is also suppressed.

Therefore, regardless of whether the hydroponic nutrient solution is circulated, the hydroponic nutrient solution having a stable composition can be used in the sterilization of the hydroponic nutrient solution using the ultraviolet rays in the hydroponic cultivation. Thus, efficient plant cultivation is achieved.

When an aqueous solution containing the complex of the present invention is used for soil cultivation, plants can be suitably cultivated in alkaline soil.

### [Examples]

### 1. Production of heterocycle-containing amino acid compounds

Hereinafter, the method of producing heterocycle-containing amino acid compounds will be specifically described with reference to Examples 1-1 to 1-9.

The resulting heterocycle-containing amino acid compounds were subjected to ¹H NMR measurement. The splitting pattern is as follows.

s: singlet; d: doublet; t: triplet; q: quartet; m: multiplet, br: broad.

### Example 1-1

Produced was a hydrochloride (V1) in which R¹ represents a hydrogen atom and n is 2 in the general formula (1).

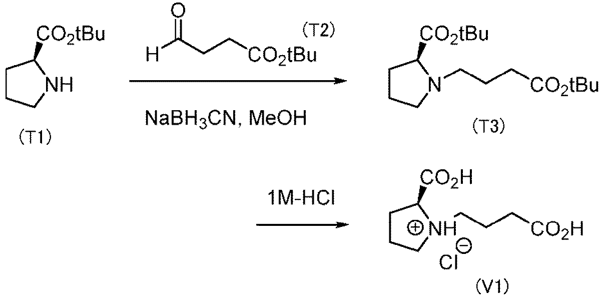

6.3 mL of a methanol solution containing 200 mg (1.26 mmol) of tert-butyl 4-oxobutanoate (T2) was cooled to a temperature of 0°C. To this solution, 216 mg (1.26 mmol) of L-proline tert-butyl (T1) and 79 mg (1.26 mmol) of sodium cyanoborohydride (NaBH₃CN) were added to react at a temperature of 0°C for 15 minutes while stirring. Then, the completion of the reaction was confirmed by TLC, a saturated aqueous sodium hydrogen carbonate solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 114 mg (0.36 mmol, 29%) of a pale yellow oily compound (T3). The developing solvent for flash column chromatography was n-hexane : ethyl acetate = 4:1 to 1:1.

Subsequently, 98 mg (0.31 mmol) of the compound (T3) was cooled to a temperature of 0°C, and 1 M HCl (3.2 mL) was added to the compound (T3). The temperature was raised to a temperature of 25°C, and the mixture was reacted for 2 hours while stirring. The completion of the reaction was confirmed by TLC, and the reaction solution was concentrated under reduced pressure to yield 72.1 mg (0.30 mmol) of a compound (V1) as a milky white solid.

### Compound (V1)

¹H NMR (400 MHz, D₂O): *δ* 4.32-4.20 (m, 1H), 3.80 (ddd, *J*=11.6, 7.4, 4.0 Hz, 1H), 3.37 (ddd, *J*=12.6, 9.8, 6.7 Hz, 1H), 3.22 (ddd, *J*=12.7, 10.1, 6.0 Hz, 2H), 2.60-2.52 (m, 1H), 2.49 (t, *J*=7.0 Hz, 2H), 2.23-2.10 (m, 2H), 2.28-1.90 (m, 3H).

### Example 1-2

Produced was a compound (V2) in which R¹ represents a hydroxy group and n is 2 in the general formula (1).

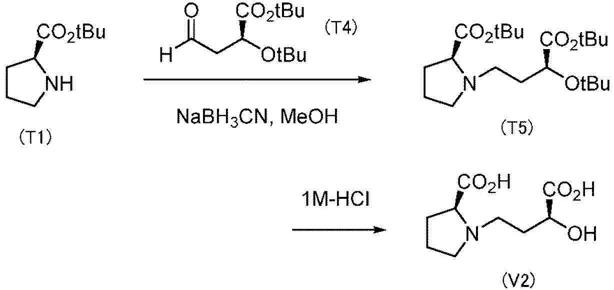

2.2 mL of a methanol solution containing 102 mg (0.44 mmol) of the aldehyde compound (T4) was cooled to a temperature of 0°C. To this solution, 75.7 mg (0.44 mmol) of L-proline tert-butyl (T1) and 28 mg (0.45 mmol) of sodium cyanoborohydride (NaBH₃CN) were added to react at a temperature of 0°C for 40 minutes while stirring. Then, the completion of the reaction was confirmed by TLC, a saturated aqueous sodium hydrogen carbonate solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 70.4 mg (0.18 mmol, 41%) of a pale yellow oily compound (T5). The developing solvent for flash column chromatography was n-hexane : ethyl acetate = 8:1 to 4:1.

Subsequently, 70.4 mg (0.18 mmol) of the compound (T5) was cooled to a temperature of 0°C, and 1 M HCl (1.8 mL) was added to the compound (T5). The temperature of the mixture was raised to a temperature of 25°C to react for 24 hours while stirring. The completion of the reaction was confirmed by TLC, and the reaction solution was concentrated under reduced pressure. The resultant residue was purified by ion exchange to yield 36.9 mg (0.17 mmol, 93%) of a compound (V2) as a milky white solid. Ion exchange was performed by combining 8 ion exchange resins "Dowex 50W" (registered trademark) manufactured by The Dow Chemical Company, with an eluate from an ion-exchanged water to 5% ammonia water.

### Compound (V2)

¹H NMR (400 MHz, D₂O): *δ* 4.09 (dd, *J*=6.6, 4.7 Hz, 1H), 3.95 (dd, *J*=9.2, 6.2 Hz, 1H), 3.75 (ddd, *J*=11.3, 7.1, 4.2 Hz, 1H), 3.38 (ddd, *J*=12.4, 10.1, 6.1 Hz, 1H), 3.24-3.08 (m, 2H), 2.50-2.38 (m, 1H), 2.18-1.88 (m, 5H).

### Example 1-3

Produced was a hydrochloride (V3) in which R¹ represents an amino group and n is 2 in the general formula (1).

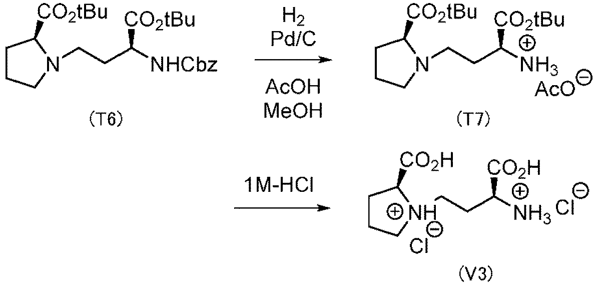

42 µL (0.73 mmol) of acetic acid and 13.3 mg of a palladium/carbon catalyst (Pd/C) were added to 1.2 mL of a methanol solution containing 112 mg (0.24 mmol) of the compound (T6). Bubbling hydrogen gas was supplied to the resultant mixture while stirring to react at a temperature of 25°C for 48 hours. Then, the completion of the reaction was confirmed by TLC, and the reaction solution was filtered through celite. The filtrate was concentrated under reduced pressure to yield 98.4 mg (0.25 mmol) of a pale yellow oily compound (T7).

Subsequently, 36.2 mg (0.093 mmol) of the compound (T7) was cooled to a temperature of 0°C, and 1 M HCl (1 mL) was added to the compound. The temperature of the mixture was raised to a temperature of 25°C to react for 24 hours while stirring. Thereafter, the reaction solution was concentrated under reduced pressure, the resultant residue was cooled to a temperature of 0°C again. 6 M HCl (1 mL) was added to the residue, and the mixture was reacted for 2 hours while stirring. The completion of the reaction was confirmed by NMR, and then the reaction solution was concentrated under reduced pressure to yield 26.1 mg (0.09 mmol) of a compound (V3) as a milky white solid.

### Compound (V3)

¹H NMR (500 MHz, D₂O): *δ* 4.37 (br t, *J*=7.3 Hz, 1H), 4.20 (dd, *J*=7.8, 5.5 Hz, 1H), 3.87 (ddd, *J*=11.3, 7.3, 4.2 Hz, 1H), 3.69 (td, *J*=11.8, 5.7 Hz, 1H), 3.45 (td, *J*=11.9, 5.3 Hz, 1H), 3.30 (br q, *J*=9.1 Hz, 1H), 2.65-2.53 (m, 1H), 2.50-2.30 (m, 2H), 2.29-2.06 (m, 2H), 2.16-1.80 (m, 1H).

### Example 1-4

Produced was a hydrochloride (V4) in which R¹ represents -NHCOCH₃ and n is 2 in the general formula (1).

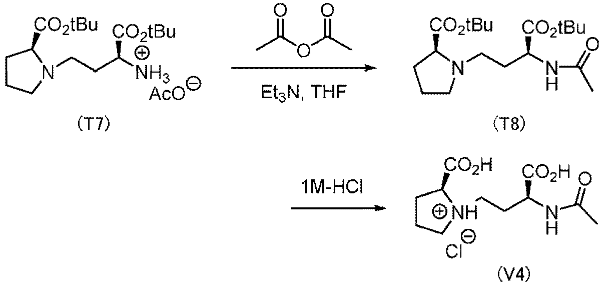

0.75 mL of a tetrahydrofuran solution containing 59.7 mg (0.15 mmol) of the compound (T7), which was an intermediate in Example 1-3, was cooled to a temperature of 0°C. To this solution, 65 µL (0.46 mmol) of triethylamine and 19 µL (0.19 mmol) of acetic anhydride were added to react at a temperature of 0°C for 90 minutes while stirring. Then, the completion of the reaction was confirmed by TLC, and the reaction solution was concentrated under reduced pressure. The resultant residue was purified by flash column chromatography to yield 34.0 mg (0.092 mmol, 60%) of a pale yellow oily compound (T8). The developing solvent for flash column chromatography was n-hexane : ethyl acetate = 2:1 to 0:1.

Subsequently, 34.1 mg (0.092 mmol) of the compound (T8) was cooled to a temperature of 0°C, and 1 M HCl (0.9 mL) was added to the compound. The temperature of the mixture was raised to a temperature of 25°C to react for 24 hours while stirring. The reaction solution was concentrated under reduced pressure to yield 32.2 mg (0.11 mmol) of a compound (V4) as a milky white solid.

### Compound (V4)

¹H NMR (400 MHz, D₂O): *δ* 4.51-4.40 (m, 1H), 4.30 (dd, *J*=9.4, 7.4 Hz, 1H), 3.86-3.72 (m, 1H), 3.46 (dt, *J*=11.8 Hz, 1H), 3.30-3.08 (m, 1H), 2.60-2.45 (m, 1H), 2.43-2.25 (m, 1H), 2.23-2.08 (m, 3H), 2.03-1.92 (m, 1H), 1.98 (s, 3H).

### Example 1-5

Produced was a compound (V5) in which R¹ represents -NHCOCH₂OH and n is 2 in the general formula (1).

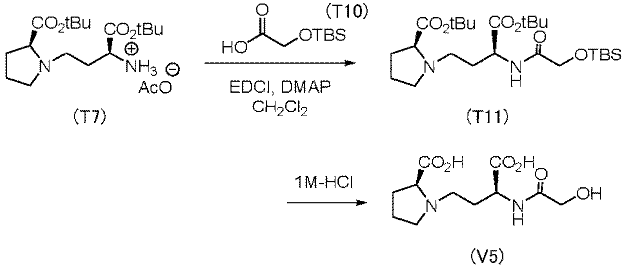

0.5 mL of a dichloromethane solution containing 39.6 mg (0.10 mmol) of the compound (T7), which was an intermediate in Example 1-3, was cooled to a temperature of 0°C. To this solution, 43.7 mg (0.23 mmol) of tert-butyldimethylsilyl glycolate, 32.2 mg (0.17 mmol) of 1-ethyl-3-(3-dimethylaminopropyl)carbodiimide hydrochloride, and 2.7 mg (0.022 mmol) of N,N-dimethyl-4-aminopyridine were added to react at a temperature of 0°C for 40 minutes while stirring. Then, the completion of the reaction was confirmed by TLC, a saturated aqueous ammonium chloride solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 25.6 mg (0.51 mmol, 50%) of a pale brown oily compound (T11). The developing solvent for flash column chromatography was n-hexane : ethyl acetate = 4:1 to 2:1.

Subsequently, 25.6 mg (0.051 mmol) of the compound (T11) was cooled to a temperature of 0°C, and 1 M HCl (0.5 mL) was added to the compound. The temperature of the mixture was raised to a temperature of 25°C to react for 24 hours while stirring. The completion of the reaction was confirmed by TLC, and the reaction solution was concentrated under reduced pressure. The resultant residue was purified by ion exchange to yield 8.1 mg (0.03 mmol, 58%) of a compound (V5) as a pale brown solid. Ion exchange was performed using an ion exchange resin "Dowex (registered trademark) 50W×2" manufactured by The Dow Chemical Company, with an eluate of 5% ammonia water.

### Compound (V5)

¹H NMR (400 MHz, D₂O): *δ* 4.29 (br t, *J*=6.5 Hz, 1H), 4.09 (d, *J*=13.8 Hz, 1H), 4.08 (d, *J*=16.8 Hz, 1H), 3.93 (dd, *J*=9.4, 6.3 Hz, 1H), 3.76 (ddd, *J*=10.6, 6.4, 4.2 Hz, 1H), 3.35 (td, *J*=11.6, 5.6 Hz, 1H), 3.20-3.07 (m, 2H), 2.51-2.38 (m, 1H), 2.32-2.19 (m, 1H), 2.18-2.00 (m, 3H), 2.00-1.80 (m, 1H).

### Example 1-6

Produced was a compound (V6) in which R¹ represents -NHCH₂COOH and n is 2 in the general formula (1).

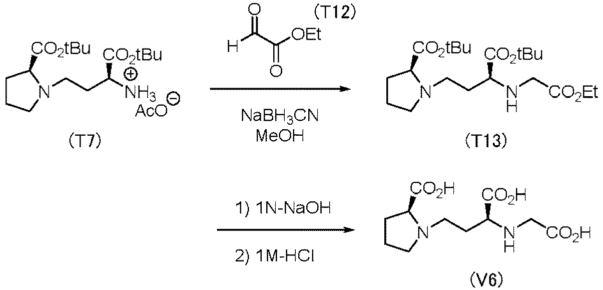

0.46 mL of a methanol solution containing 35.8 mg (0.092 mmol) of the compound (T7), which was an intermediate in Example 1-3, was cooled to a temperature of 0°C. To this solution, 20 µL (0.095 mmol) of a 47% toluene solution containing ethyl glyoxylate (T12) and 6.3 mg (0.10 mmol) of sodium cyanoborohydride (NaBH₃CN) were added, and the temperature was raised to a temperature of 25°C to react for 24 hours while stirring. The completion of the reaction was confirmed by TLC, a saturated aqueous sodium hydrogen carbonate solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 18.7 mg (0.045 mmol, 50%) of a pale yellow oily compound (T13). The developing solvent for flash column chromatography was n-hexane : ethyl acetate = 4:1 to 1:1.

Subsequently, 0.2 mL of a tetrahydrofuran solution containing 18.7 mg (0.045 mmol) of the compound (T13) was cooled to a temperature of 0°C. 1 M sodium hydroxide (53 µL) was added to the solution, and the temperature of the mixture was raised to a temperature of 25°C to react for 24 hours while stirring. The completion of the reaction was confirmed by TLC, and the reaction solution was concentrated under reduced pressure. The resultant residue was cooled to a temperature of 0°C, and 1 M hydrochloric acid (0.44 mL) was added to the residue. The temperature was raised to a temperature of 25°C to react for 48 hours while stirring. The completion of the reaction was confirmed by TLC, and the reaction solution was concentrated under reduced pressure. The resultant residue was purified by ion exchange to yield 10.2 mg (0.037 mmol, 83%) of a compound (V6) as a pale yellow solid. Ion exchange was performed using an ion exchange resin "Dowex (registered trademark) 50W×2" manufactured by The Dow Chemical Company, with an eluate of 5% ammonia water.

### Compound (V6)

¹H NMR (400 MHz, D₂O): *δ* 3.97 (dd, *J*=9.2, 6.3 Hz, 1H), 3.79 (dd, *J*=7.7, 4.6 Hz, 1H), 3.65 (d, *J*=16.1 Hz, 1H), 3.62 (d, *J*=16.1 Hz, 1H), 3.35-3.42 (m, 1H), 3.33-3.29 (m, 1H), 3.14 (br q, *J*=10.3 Hz, 1H), 2.53-2.39 (m, 1H), 2.37-2.23 (m, 2H), 2.18-2.02 (m, 2H), 2.02-1.87 (m, 1H).

### Example 1-7

Produced was a compound (V7) in which R¹ represents -N(CH₃)COCH₂OH and n is 2 in the general formula (1).

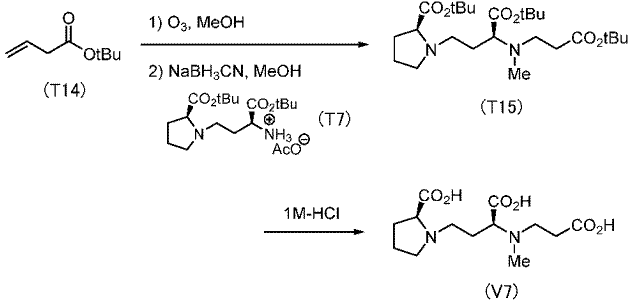

0.31 mL of a methanol solution containing 44.3 mg (0.31 mmol) of tert-butyl-3-butenoate (T14) was cooled to a temperature of -78°C. Bubbling ozone gas was supplied to the solution until the solution became blue. Then, bubbling nitrogen gas was supplied until the blue color of the solution disappeared. Thereafter, to this solution, 98 mg (0.25 mmol) of the compound (T7), which was an intermediate in Example 1-3, and 22.5 mg (0.36 mmol) of sodium cyanoborohydride (NaBH₃CN) were added, and the temperature was raised to a temperature of 25°C to react for 24 hours while stirring. The completion of the reaction was confirmed by TLC, a saturated aqueous sodium hydrogen carbonate solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 32.3 mg (0.069 mmol, 22%) of a pale green oily compound (T15). The developing solvent for flash column chromatography was n-hexane : ethyl acetate = 6:1 to 2:1.

Subsequently, 32.3 mg (0.069 mmol) of the compound (T15) was cooled to a temperature of 0°C, and 1 M hydrochloric acid (0.7 mL) was added to the compound. The temperature of the mixture was raised to a temperature of 25°C to react for 24 hours while stirring. The completion of the reaction was confirmed by TLC, and the reaction solution was concentrated under reduced pressure. The resultant residue was cooled to a temperature of 0°C, and 1 M hydrochloric acid (0.44 mL) was added to the residue. The temperature was raised to a temperature of 25°C to react for 48 hours while stirring. The completion of the reaction was confirmed by TLC, and the reaction solution was concentrated under reduced pressure. The resultant residue was purified by ion exchange to yield 18.3 mg (0.061 mmol, 88%) of a compound (V7) as a milky white solid. Ion exchange was performed using an ion exchange resin "Dowex (registered trademark) 50W×2" manufactured by The Dow Chemical Company, with an eluate of 5% ammonia water.

### Compound (V7)

¹H NMR (400 MHz, D₂O): *δ* 3.97 (br t, *J*=7.7 Hz, 1H), 3.88 (d, *J*=9.3 Hz, 1H), 3.85-3.76 (m, 1H), 3.56-3.45 (m, 1H), 3.45-3.34 (m, 3H), 3.14 (br q, *J*=9.0 Hz, 1H), 2.82 (s, 3H), 2.67-2.60 (br t, *J*=6.1 Hz, 2H), 2.53-2.49 (m, 1H), 2.36-2.20 (m, 2H), 2.20-2.03 (m, 2H), 2.01-1.86 (m, 1H).

### Example 1-8

Produced was a compound (V8) in which R¹ represents -NH₂ and n is 1 in the general formula (1).

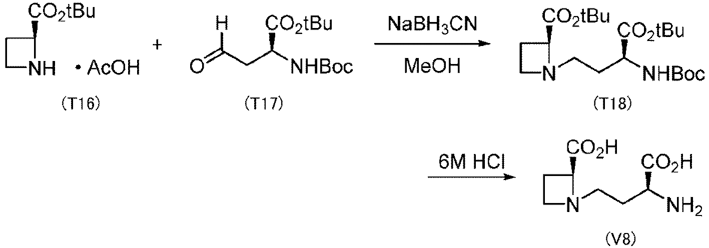

A methanol solution containing 277 mg (1.01 mmol) of the aldehyde compound (T17) was cooled to a temperature of 0°C. To this solution, 222 mg (1.02 mmol) of L-tBu azetidine (T16) and 71 mg (1.11 mmol) of sodium cyanoborohydride (NaBH₃CN) were added to react at a temperature of 0°C while stirring. Then, the completion of the reaction was confirmed by TLC, a saturated aqueous sodium hydrogen carbonate solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 308 mg (0.74 mmol, 73%) of a pale yellow oily compound (T18). The developing solvent for flash column chromatography was n-hexane : ethyl acetate = 4:1.

Subsequently, 101 mg (0.24 mmol) of the compound (T18) was cooled to a temperature of 0°C, and 6 M HCl (2.5 mL) was added to the compound. The temperature of the mixture was raised to a temperature of 30°C to react for 1 hour while stirring at a reduced pressure of 400 Torr. Thereafter, the reaction solution was concentrated under reduced pressure, and the residue was purified by ion exchange to yield 44 mg (0.22 mmol, 90%) of a compound (V8) as a milky white solid. Ion exchange was performed using an ion exchange resin "Amberlite^{®} IRC-120(H)" (registered trademark) manufactured by Thermo Scientific Chemicals, with an eluate of 5% ammonia water.

### Compound (V8)

¹H NMR (500 MHz, D₂O): *δ* 4.68 (t, *J*=9.4 Hz, 1H), 4.01 (br t, *J*=9.5 Hz, 1H), 3.86 (q, *J*=9.6 Hz, 1H), 3.78 (t, *J*=6.3 Hz, 1H), 3.43-3.37 (m, 1H), 3.32-3.24 (m, 1H), 2.66 (br q, *J*=10.6 Hz, 1H), 2.52-2.41 (m, 1H), 2.11-2.04 (m, 2H).

### Example 1-9

Produced was a compound (V9) in which R¹ represents -OH and n is 1 in the general formula (1).

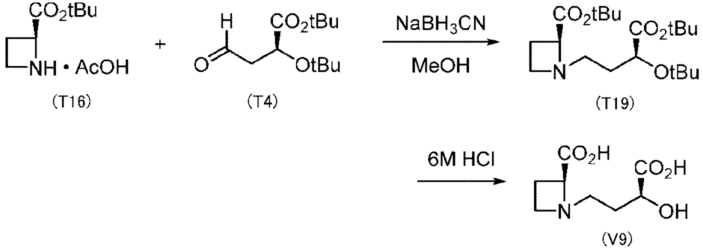

A methanol solution containing 250 mg (1.08 mmol) of the aldehyde compound (T4) was cooled to a temperature of 0°C. To this solution, 259 mg (1.12 mmol) of L-tBu azetidine (T16) and 76 mg (1.19 mmol) of sodium cyanoborohydride (NaBH₃CN) were added to react at a temperature of 0°C for 70 minutes while stirring. Then, the completion of the reaction was confirmed by TLC, a saturated aqueous sodium hydrogen carbonate solution was added to this reaction solution. Further, ethyl acetate was added to the resultant mixed solution for solvent extraction. The organic layer was collected and dried over magnesium sulfate. Thereafter, filtration was performed, and the filtrate was concentrated under reduced pressure. The resultant residue was purified by flash column chromatography to yield 226 mg (0.61 mmol, 56%) of a compound (T19) as a white crystal. The developing solvent for flash column chromatography was n-hexane : ethyl acetate = 6:1.

Subsequently, 114 mg (0.31 mmol) of the compound (T19) was cooled to a temperature of 0°C, and 6 M HCl (3 mL) was added to the compound. The temperature of the mixture was raised to a temperature of 30°C to react for 1 hour while stirring at a reduced pressure of 400 Torr. Thereafter, the reaction solution was concentrated under reduced pressure, and the residue was purified by ion exchange to yield 65 mg (0.29 mmol, 96%) of a compound (V9) as a milky white solid. Ion exchange was performed using an ion exchange resin "Amberlite^{®} IRC-120(H)" (registered trademark) manufactured by Thermo Scientific Chemicals, with an eluate of 5% ammonia water.

### Compound (V9)

¹H NMR (500 MHz, D₂O): *δ* 4.65 (t, *J*=9.5 Hz, 1H), 4.05 (dd, *J*=7.0, 4.6 Hz, 1H), 4.02 (td, *J*=9.8, 4.5 Hz, 1H), 3.86 (q, *J*=9.5 Hz, 1H), 3.34 (ddd, *J*=12.7, 9.8, 6.2 Hz, 1H), 3.17 (ddd, *J*=12.7, 10.0, 5.1 Hz, 1H), 2.66 (dtd, *J*=12.1, 9.6, 4.5 Hz, 1H), 2.44 (dq, *J*=11.8, 9.4 Hz, 1H), 2.02-1.93 (m, 1H), 1.90-1.80 (m, 1H).

### 2. Production of complexes using heterocycle-containing amino acid compounds, and complex evaluation (1)

Complexes were produced using the heterocycle-containing amino acid compounds prepared in Examples 1-1 to 1-9, and an aqueous solution of each complex was irradiated with ultraviolet rays to measure the absorbance. For comparison, absorbance measurement was performed using an aqueous solution of each of an iron complex of ethylenediaminetetraacetic acid (EDTA) (hereinafter, referred to as "Fe-EDTA"), an iron complex of diethylenetriamine-N,N,N',N",N"-pentaacetic acid (DTPA) (hereinafter, referred to as "Fe-DTPA"), an iron complex of hydroxybenzylethylenediamine (HBED) (hereinafter, referred to as "Fe-HBED"), an iron complex of N,N'-ethylene-bis(hydroxyphenyl)glycine (EDDHA) (hereinafter, referred to as "Fe-EDDHA"), an iron complex of citric acid, and an iron complex (hereinafter, referred to as "Fe-PDMA") of a compound represented by the following formula (hereinafter, referred to as "PDMA"). The concentration of Fe in each of the aqueous solutions for absorbance measurement is 100 µM or 200 µM (dimer).

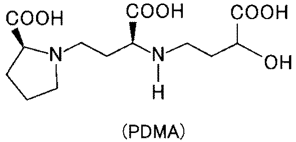

### Example 2-1

Approximately 5.2 milligrams of the compound (V1) was dissolved in 200 mL of a deionized water. 5.5 mL of an aqueous solution containing 4.0 mM iron (III) sulfate was added to this aqueous solution, and the pH was adjusted to 5.0. Subsequently, a deionized water was added to fill up to 220 mL. This aqueous solution was left to stand at a temperature of 25°C for 1 hour to yield an aqueous solution of an iron complex having an Fe concentration of 100 µM (5.6 ppm). Thereafter, the absorbance of this aqueous complex solution was measured.

The absorbance was measured by placing the aqueous complex solution in a 10-mm-wide cell, and irradiating the cell with ultraviolet rays having a wavelength of 253.7 nm using an ultraviolet absorbance measuring device "UV-mini 1240" (model name) manufactured by Shimadzu Corporation (see Table 1).

### Example 2-2

Approximately 10.4 milligrams of the compound (V1) was dissolved in 200 mL of a deionized water. 5.5 mL of an aqueous solution containing 4.0 mM iron (III) sulfate was added to this aqueous solution, and the pH was adjusted to 5.0. Subsequently, a deionized water was added to fill up to 220 mL. This aqueous solution was left to stand at a temperature of 25°C for 1 hour to yield an aqueous solution of a dimer complex having an Fe concentration of 100 µM and containing two molecules of the compound (V1) coordinated to one Fe ion. Thereafter, the absorbance of this aqueous complex solution was measured in the same manner as described above (see Table 1).

### Examples 2-3 to 2-5

Aqueous complex solutions were prepared in the same manner as those in Example 2-1 except that the compounds (V2) to (V4) were used in place of the compound (V1), and absorbances were measured (see Table 1).

### Example 2-6

An aqueous solution of a dimer complex having an Fe concentration of 100 µM and containing two molecules of the compound (V4) coordinated to one Fe ion was prepared in the same manner as those in Example 2-2 except that the compound (V4) was used in place of the compound (V1), and the absorbance was measured (see Table 1).

### Examples 2-7 to 2-10

Aqueous complex solutions were prepared in the same manner as those in Example 2-1 except that the compounds (V5) to (V8) were used in place of the compound (V1), and absorbances were measured (see Table 1).

### Example 2-11

An aqueous solution of a dimer complex having an Fe concentration of 100 µM and containing two molecules of the compound (V8) coordinated to one Fe ion was prepared in the same manner as those in Example 2-2 except that the compound (V8) was used in place of the compound (V1), and the absorbance was measured (see Table 1).

### Example 2-12

An aqueous complex solution was prepared in the same manner as those in Example 2-1 except that the compound (V9) was used in place of the compound (V1), and the absorbance was measured (see Table 1).

### Example 2-13

An aqueous solution of a dimer complex having an Fe concentration of 100 µM and containing two molecules of the compound (V9) coordinated to one Fe ion was prepared in the same manner as those in Example 2-2 except that the compound (V9) was used in place of the compound (V1), and the absorbance was measured (see Table 1).

### Comparative Example 2-1

An aqueous solution of an iron complex of citric acid (hereinafter, also referred to as "Fe-citric acid") was prepared in the same manner as those in Example 2-1 except that citric acid was used in place of the compound (V1), and the absorbance was measured (see Table 1).

### Comparative Example 2-2

An aqueous solution of a dimer complex having an Fe concentration of 100 µM and containing two molecules of citric acid coordinated to one Fe ion (hereinafter, also referred to as "Fe-citric acid × 2") was prepared in the same manner as those in Example 2-2 except that citric acid was used in place of the compound (V1), and the absorbance was measured (see Table 1).

### Comparative Example 2-3

An aqueous solution of Fe-PDMA was prepared in the same manner as those in Example 2-1 except that PDMA was used in place of the compound (V1), and the absorbance was measured (see Table 1).

### Comparative Example 2-4

Fe-EDTA powder was dissolved in 200 mL of a deionized water and then the pH of the aqueous solution was adjusted to 5.0 using NaOH. Subsequently, about 220 mL of a deionized water was added to the resultant mixture to yield an aqueous complex solution having an Fe concentration of 100 µM (5.6 ppm). Thereafter, the absorbance was measured in the same manner as in Example 2-1 (see Table 1).

### Comparative Examples 2-5 to 2-7

Aqueous complex solutions were prepared in the same manner as those in Comparative Example 2-4 except that Fe-DTPA, Fe-EDDHA, or Fe-HBED was used in place of Fe-EDTA. Thereafter, absorbances were measured in the same manner as in Example 2-1 (see Table 1).

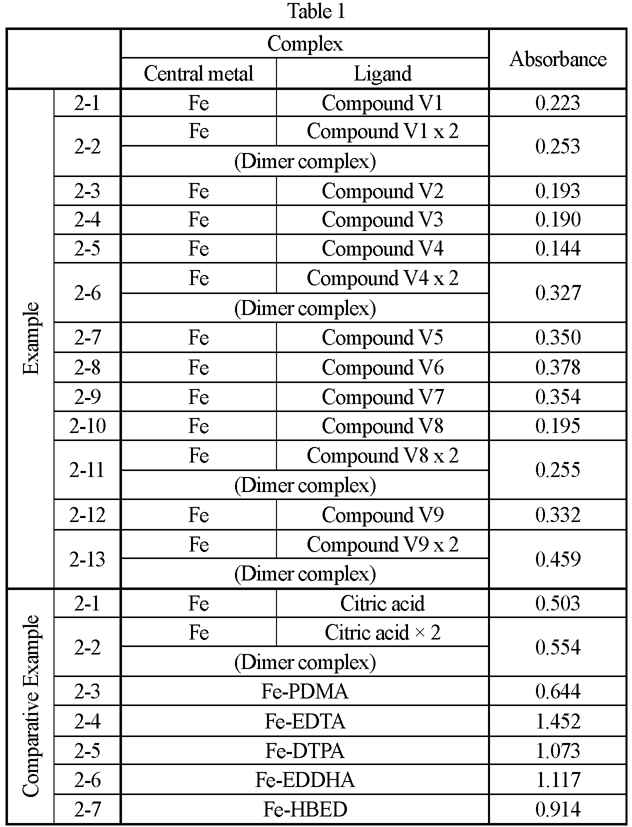

Examples 2-1 to 2-13 are examples of complexes prepared using the heterocycle-containing amino acid compound of the present invention. When aqueous solutions of these complexes were irradiated with ultraviolet rays, absorbances were less than 0.500, which were lower than absorbances of the aqueous complex solutions obtained in Comparative Examples 2-1 to 2-7. Accordingly, it can be seen that the complex of the present invention is highly stable in an aqueous solution irradiated with ultraviolet rays.

### 3. Complex evaluation (2)

Each of the complexes obtained in Comparative Examples 2-2 and 2-5 was added to a liquid based on the formulation in the Horticultural Experiment Station. The pH of the mixture was adjusted to 5.0 using NaOH to prepare 1.5 L of a nutrient solution having an Fe concentration of 100 µM.

Subsequently, the nutrient solution was continuously irradiated with ultraviolet rays having a wavelength of 253.7 nm using a sterilization lamp "Turbo Twist 3X 9W" (trade name) manufactured by KAMIHATA FISH INDUSTRIES LTD., while circulating the nutrient solution. The Fe concentrations in the nutrient solution after 15 minutes, 30 minutes, 60 minutes, and 90 minutes were measured using a simple reflection photometer "RQflex" (trade name) manufactured by Merck & Co., Inc. (see Table 2).

**Table 2**

| Ultraviolet irradiation time (min) | Fe concentration (mg/L) | |
|---|---|---|
| | Comparative Example 2-2 (Fe-citric acid × 2) | Comparative Example 2-5 (Fe-DTPA) |
| 0 | 5.2 | 4.8 |
| 15 | 5.2 | 4.8 |
| 30 | 4.9 | 0.6 |
| 60 | 2.6 | 0 |
| 90 | 0 | 0 |

### 4. Plant cultivation test (1)

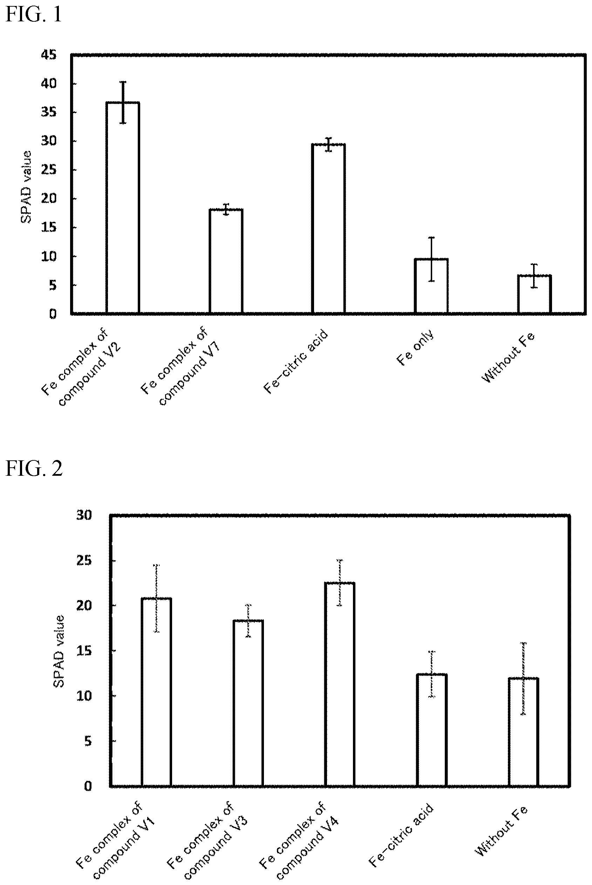

Seeds of corn variety "Launcher 82" were placed on a paper towel wetted with tap water. 8 days after germination, the seedlings were immersed in 45 mL of a hydroponic nutrient solution (a liquid based on the formulation in the Horticultural Experiment Station). 2 days after that, 3 mL of iron materials including various aqueous complex solutions were administered to the seedlings. Then, on the 3rd day after the administration of the iron materials, the leaf color (SPAD value) of corn was measured. This hydroponic cultivation was performed in two pots, and the average value of the SPAD values was calculated. For comparison, an experiment of administering only iron sulfate and an experiment of administering no iron material were also performed. These results are shown in Table 3 and Fig. 1.

Here, methods of preparing the iron materials will be described.

Iron materials containing the Fe complex of the compound (V2) or citric acid are aqueous complex solutions having an Fe concentration of 100 µM, the aqueous complex solutions being obtained by preparing a first aqueous solution having a concentration of the compound (V2) or citric acid of 0.2 mM using a deionized water, and then adding iron sulfate to the first aqueous solution and dissolving therein to have an iron sulfate concentration of 0.1 mM.

An iron material containing the Fe complex of the compound (V7) is an aqueous complex solution having an Fe concentration of 100 µM, the aqueous complex solution being obtained by preparing a first aqueous solution having a concentration of the compound (V7) of 0.1 mM using a deionized water, and then adding iron sulfate to the first aqueous solution and dissolving therein to have an iron sulfate concentration of 0.1 mM.

**Table 3**

| | SPAD value | |
|---|---|---|
| | Average | Standard deviation |
| Fe complex of compound V2 | 36.7 | 5.1 |
| Fe complex of compound V7 | 18.1 | 1.3 |
| Fe-citric acid | 29.4 | 1.6 |
| Fe only | 9.5 | 5.4 |
| Without Fe | 6.6 | 2.8 |

### 5. Plant cultivation test (2)

Seeds of paddy rice variety "Nipponbare" were seeded in tap water, and soaked at a temperature of 27°C for 7 days to germinate. Thereafter, the germinated seeds were immersed in a hydroponic nutrient solution (a liquid based on the formulation in the Horticultural Experiment Station). After 7 days, 3 seedlings as one plant were transplanted into soil obtained by adding fertilizer to 800 g of shelly fossil soil with pH9 per pot, and soil cultivation was started. The soil cultivation was performed at a temperature of 28°C for 14 hours in the day and at 23°C for 10 hours in the night. The soil was water-sprinkled once every 2 days such that the amount of water in the soil was 270 mL. After 7 days from the start of soil cultivation, 40 mL of the various iron material including the aqueous complex solution was administered such that the amount of water in the soil was 270 mL. Then, the rice was water-sprinkled once every 3 days. On the 6th day after the administration of the iron materials, the leaf color (SPAD value) of rice was measured This soil cultivation was performed in 3 pots, and the average value of the SPAD values was calculated. For comparison, an experiment of administering no iron material was also performed. These results are shown in Table 4 and Fig. 2.

Here, methods of preparing the iron materials will be described.

Iron materials containing the Fe complex of the compound (V1) or citric acid are aqueous complex solutions having an Fe concentration of 600 µM, the aqueous complex solutions being obtained by preparing a first aqueous solution having a concentration of the compound (V1) or citric acid of 1.2 mM using a deionized water, and then adding iron sulfate to the first aqueous solution and dissolving therein to have an iron sulfate concentration of 0.6 mM.

Iron materials containing the Fe complex of the compound (V3) or the compound (V4) are aqueous complex solutions having an Fe concentration of 600 µM, the aqueous complex solutions being obtained by preparing a first aqueous solution having a concentration of the compound (V3) or the compound (V4) of 0.6 mM using a deionized water, and then adding iron sulfate to the first aqueous solution and dissolving therein to have an iron sulfate concentration of 0.6 mM.

**Table 4**

| | SPAD value | |
|---|---|---|
| | Average | Standard deviation |
| Fe complex of compound V1 | 20.8 | 3.7 |
| Fe complex of compound V3 | 18.3 | 1.8 |
| Fe complex of compound V4 | 22.5 | 2.5 |
| Fe-citric acid | 12.4 | 2.5 |
| Without Fe | 11.9 | 3.9 |

### 6. Plant cultivation test (3)

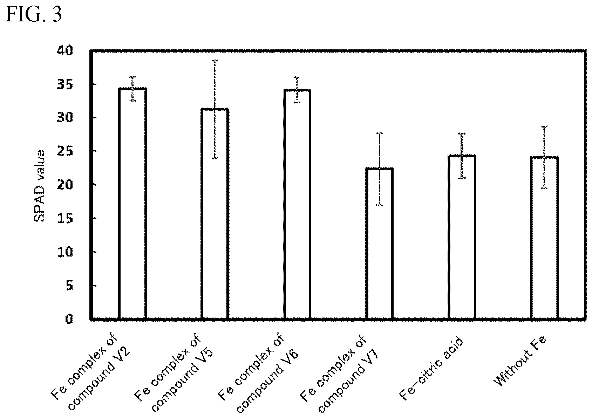

Seeds of paddy rice variety "Nipponbare" were seeded in tap water, and soaked at a temperature of 27°C for 7 days to germinate. Thereafter, the germinated seeds were immersed in a hydroponic nutrient solution (a liquid based on the formulation in the Horticultural Experiment Station). After 6 days, 3 seedlings as one plant were transplanted into soil obtained by adding fertilizer to 800 g of shelly fossil soil with pH9 per pot, and soil cultivation was started. The soil cultivation was performed at a temperature of 28°C for 14 hours in the day and at 23°C for 10 hours in the night. The soil was water-sprinkled once every 2 days such that the amount of water in the soil was 270 mL. After 7 days from the start of soil cultivation, 40 mL of the various iron material including the aqueous complex solution was administered such that the amount of water in the soil was 270 mL. Then, the rice was water-sprinkled once every 3 days. On the 6th day after the administration of the iron materials, the leaf color (SPAD value) of rice was measured This soil cultivation was performed in 3 pots, and the average value of the SPAD values was calculated. For comparison, an experiment of administering no iron material was also performed. These results are shown in Table 5 and Fig. 3.

Here, methods of preparing the iron materials will be described.

Iron materials containing the Fe complex of the compound (V2) or citric acid are aqueous complex solutions having an Fe concentration of 200 µM, the aqueous complex solutions being obtained by preparing a first aqueous solution having a concentration of the compound (V2) or citric acid of 0.4 mM using a deionized water, and then adding iron sulfate to the first aqueous solution and dissolving therein to have an iron sulfate concentration of 0.2 mM.

Iron materials containing the Fe complex of the compound (V5), Fe complex of the compound (V6), or Fe complex of the compound (V7) are aqueous complex solutions having an Fe concentration of 200 µM, the aqueous complex solutions being obtained by preparing a first aqueous solution having a concentration of the compound (V5), Fe complex of the compound (V6), or Fe complex of the compound (V7) of 0.2 mM using a deionized water, and then adding iron sulfate to the first aqueous solution and dissolving therein to have an iron sulfate concentration of 0.2 mM.

**Table 5**

| | SPAD value | |
|---|---|---|
| | Average | Standard deviation |
| Fe complex of compound V2 | 34.3 | 1.8 |
| Fe complex of compound V5 | 31.3 | 7.3 |
| Fe complex of compound V6 | 34.2 | 1.9 |
| Fe complex of compound V7 | 22.4 | 5.4 |
| Fe-citric acid | 24.3 | 3.3 |
| Without Fe | 24.1 | 4.6 |

### [Industrial Applicability]

When a solution of the complex formed using the heterocycle-containing amino acid compound of the present invention is irradiated with ultraviolet rays, decomposition of the complex is suppressed. Taking advantage of the property, the complex can be suitably used as a raw material of, for example, a hydroponic nutrient solution to be sterilized by ultraviolet irradiation. Particularly, when the metal element contained in the complex is Fe, the complex is suitable as a raw material of a hydroponic nutrient solution suitable for plant cultivation.

## Claims

1. A heterocycle-containing amino acid compound represented by a general formula (1) or a salt thereof, 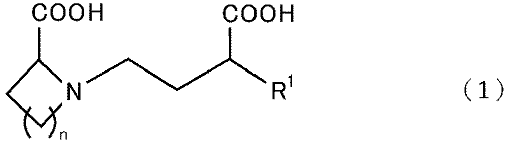 wherein n is 1, 2, or 3, and R¹ represents a hydrogen atom, -OH, -NH₂, -NH-CO-CH₃, -NH-CO-CH₂OH, or -NR²-(CH₂)ₘ-COOH, where m is 1 or 2, and R² represents a hydrogen atom or a methyl group.

2. The heterocycle-containing amino acid compound or a salt thereof according to claim 1, wherein the heterocycle-containing amino acid compound is a compound represented by a general formula (2) or (3), 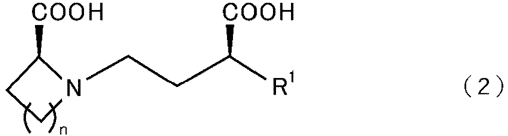 wherein n is 1, 2, or 3, and R¹ represents -OH, -NH₂, -NH-CO-CH₃, -NH-CO-CH₂OH, or -NR²-(CH₂)ₘ-COOH, where m is 1 or 2, and R² represents a hydrogen atom or a methyl group, 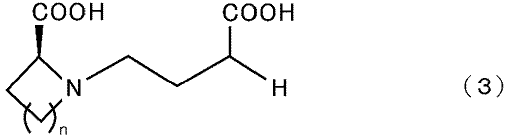 wherein n is 1, 2 or 3.

3. A complex comprising: the heterocycle-containing amino acid compound or a salt thereof according to claim 1 or 2; and a metal element.

4. The complex according to claim 3, wherein the metal element is Fe.
